# EUROPÄISCHE PATENTSCHRIFT

(11) **EP 1 970 083 B1**
(45) Veröffentlichungstag und Bekanntmachung des Hinweises auf die Patenterteilung: **10.05.2017**
(21) Anmeldenummer: 07005215.4
(22) Anmeldetag: 14.03.2007
(51) Int. Cl.: A61M 5/158

(54) **Insertionsvorrichtung für einen Insertionskopf, insbesondere für ein Infusionsset**
Insertion device for an insertion head, in particular for an infusion set
Dispositif d'insertion pour une tête d'insertion, en particulier pour un ensemble d'infusion

(43) Veröffentlichungstag der Anmeldung: 17.09.2008
(73) Patentinhaber: F. Hoffmann-La Roche AG, 4070 Basel (CH); Roche Diabetes Care GmbH, 68305 Mannheim (DE)
(72) Erfinder: Liniger, Jürg, 3072 Ostermundingen (CH); Rütti, Pascal, 4623 Neuendorf (CH); Collins, James, Peterbourough Cambridgeshire PE73LW (GB)
(74) Vertreter: Rentsch Partner AG

(56) Entgegenhaltungen:
- EP-A- 1 652 547
- WO-A-2005/037184
- WO-A1-2006/077262
- US-A- 5 848 990

## Beschreibung

Die vorliegende Erfindung betrifft eine Insertionsvorrichtung für einen Insertionskopf sowie eine Anordnung umfassend die Insertionsvorrichtung und einen in dieser aufgenommenen oder aufnehmbaren Insertionskopf, gemäss den Oberbegriffen der unabhängigen Patentansprüche.

Bei Patienten, welche einen regelmässigen Bedarf an einem durch direkte Zuführung in das Körpergewebe oder in den Blutkreislauf verabreichbaren Medikament haben, wie z.B. bestimmte Gruppen von Schmerzpatienten oder Patienten mit Diabetes Typ I und Typ II, kann es sinnvoll sein, dem Körper die erforderliche Medikamentenmenge in flüssiger Form über eine an geeigneter Stelle in den Körper eingebrachte und über einen längeren Zeitraum dort verbleibende Kanüle zuzuführen. Hierzu wird auf der Haut des Patienten eine je nach Ausführung als "Infusionsset" oder "Port" bezeichnete Anordnung mit einer Kanüle befestigt, derart, dass die Kanüle durch die Haut in den Körper eindringt.

Auch gibt es zunehmend Bestrebungen, bestimmte medizinische Parameter eines Patienten, wie z.B. den Blutzuckerwert, über einen längeren Zeitraum kontinuierlich zu überwachen. Hierzu wird beispielsweise am Körper des Patienten eine Sensoranordnung angeordnet, welche mit einer Einstechspitze eines geeigneten Sensors durch die Haut in den Körper des Patienten eindringt.

Um Infektionen zu vermeiden, muss das Infusionsset, der Port oder die Sensoranordnung in regelmässigen Zeitintervallen gewechselt werden, z.B. alle drei Tage, was bei der nicht stationären Behandlung, z.B. von Diabetikern, oftmals durch den Patienten selbst erfolgt und infolge des Einstechens der Infusionskanüle oder der Einstechspitze mit gewissen Schmerzen verbunden ist. Entsprechend wichtig ist es, dass solche Infusionssets, Ports oder Sensoranordnungen einfach und sicher zu applizieren sind, weshalb viele Hersteller inzwischen dazu übergegangen sind, ihre Produkte als Insertionsköpfe für spezielle Insertionsvorrichtungen auszubilden, mit Hilfe welcher diese am Körper des Patienten appliziert werden können. Hierdurch wird das Applizieren erleichtert und der Applikationsschmerz infolge des schnellen und gezielten Einstechens auf ein Minimum reduziert.

So sind z.B. aus US 6,607,509 B2, EP 1 652 547 A1 und WO 2006/077262 A1 Insertionsvorrichtungen für Infusionssets bekannt, bei denen das Infusionsset durch die Kraft einer vorgespannten Feder schlagartig auf die Applikationsstelle aufgesetzt wird, unter einem Einstechen der Kanüle in das Gewebe des Patienten. Nach erfolgter Applikation des Infusionssets muss die Insertionsvorrichtung vom Infusionsset abgekoppelt und entfernt werden, was den Nachteil birgt, dass es hierbei zu einer Irritation der Einstechstelle durch Kraftausübung auf die eingestochene Kanüle kommen kann.

WO 2004/110527 A1 offenbart neben Insertionsvorrichtungen wie zuvor beschrieben ähnliche Insertionsvorrichtungen für Infusionssets, bei denen das Infusionsset jedoch bereits beim Einstechen in den Körper des Patienten automatisch von der Insertionsvorrichtung getrennt wird. Hierdurch ergibt sich gegenüber den zuvor beschriebenen Insertionsvorrichtungen der Vorteil, dass nur geringe Reibungsverluste innerhalb der Insertionsvorrichtung entstehen, so dass eine schlagartige Applikation mit einer entsprechend kurzen Schmerzphase möglich wird und zudem eine Irritation der Einstechstelle durch ein nachträgliches Lösen der Insertionsvorrichtung vom Insertionskopf vermieden wird. Diese Insertionsvorrichtungen weisen jedoch den Nachteil auf, dass sie relativ kompliziert in der Anwendung sind und viele Bedienungsschritte erforderlich sind.

WO 2006/077262 A1 offenbart eine zur Befestigung auf der Haut eines Patienten vorgesehene Infusionsvorrichtung. Diese weist eine federkraftgetriebene Insertionsvorrichtung mit einer durch ein Rückhaltelement zunächst in einer gespannten Ausgangslage gehaltenen Kanüle auf. Durch einen Auslösevorgang wird die Rückhaltung aufgelöst und die Kanüle durch die Federkraft in die Haut eingestochen, wobei sie eine zwangsgeführte Vortriebsbewegung ausführt.

Es stellt sich daher die Aufgabe, eine Insertionsvorrichtung, eine Anordnung umfassend eine Insertionsvorrichtung mit einem zugehörigen Insertionskopf sowie ein Verfahren zum Applizieren eines Insertionskopfes zur Verfügung zu stellen, welche die Nachteile des Standes der Technik nicht aufweisen oder diese zumindest teilweise vermeiden.

Diese Aufgabe wird durch die Insertionsvorrichtung gemäss Patentanspruch 1 und die Anordnung gemäss Patentanspruch 28 gelöst.

Demgemäss betrifft ein erster Aspekt der Erfindung eine Insertionsvorrichtung für einen Insertionskopf mit mindestens einer Infusionskanüle und/oder mindestens einer Einstechspitze. Ein mit der Insertionsvorrichtung zu applizierender Insertionskopf kann also z.B. eine einzelne Infusionskanüle oder eine einzelne Einstechspitze aufweisen, mehrere Kanülen oder mehrere Einstechspitzen aufweisen oder auch eine oder mehrere Kanülen und eine oder mehrere Einstechspitzen aufweisen und des Weiteren z.B. als Infusionsset, als Port und/oder als eine Sensoranordnung, z.B. zur Messung des Blutzuckerwertes, ausgebildet sein. Die Insertionsvorrichtung weist ein Gehäuse mit einer oder mehreren Kontaktflächen zum Aufsetzen derselben auf die Haut des Patienten beim Applizieren des Insertionskopfes auf. Weiter umfasst die Insertionsvorrichtung Haltemittel, z.B. zwei sich gegenüberliegende Blattfederelemente bzw. Federlaschen, mit denen der zu applizierende Insertionskopf vorübergehend an der Insertionsvorrichtung gehalten werden kann, so dass beim eigentlichen Applizieren des Insertionskopfes lediglich die Insertionsvorrichtung vom Bediener an der Applikationsstelle gehalten werden muss. Auch verfügt die Insertionsvorrichtung über Antriebsmittel, mit denen der zu applizierende Insertionskopf bei applikationsbereiter Insertionsvorrichtung von einer ersten Position, in welcher der Insertionskopf derartig mit den Haltemitteln ge halten ist, dass sämtliche seiner Infusionskanülen und Einstechspitzen zur Vermeidung eines unbeabsichtigten Inkontaktkommens mit dem Bediener gegenüber der oder den Kontaktflächen zurückstehen, relativ zu der Kontaktfläche in Längsrichtung einer der Infusionskanülen oder Einstechspitzen in eine zweite Position, in welcher sämtliche seiner Infusionskanülen und Einstechspitzen im wesentlichen vollständig über die Kontaktfläche hinausstehen, bewegt werden kann, zur Ermöglichung eines Einstechens dieser Infusionskanülen und Einstechspitzen in den Körper des Patienten bei mit der Kontaktfläche auf der Haut des Patienten aufgesetzter Insertionsvorrichtung. Diese Bewegung des Insertionskopfes, mit welcher das eigentliche Applizieren des Insertionskopfes am Körper erfolgt, wird anspruchsgemäss als Insertionsbewegung bezeichnet.

Das Gehäuse weist einen ersten und einen zweiten Gehäuseteil auf, wobei der erste Gehäuseteil die Haltemittel und die Kontaktfläche bzw. bei mehreren Kontaktflächen einen Teil der Kontaktflächen trägt. Der zweite Gehäuseteil ist gegenüber dem ersten Gehäuseteil bewegbar, und zwar von einer Grundposition, in welcher die beiden Gehäuseteile derartig zueinander positioniert sind, wie dies bei der Applikation des Insertionskopfes am Körper des Patienten der Fall ist und in welcher ein Einsetzen des zu applizierenden Insertionskopfes in die Haltemittel typischerweise schlecht oder nicht möglich ist, in eine Ladeposition, in welcher die Haltemittel zugänglich sind, insbesondere deutlich besser zugänglich sind als in der Grundposition, zum bestimmungsgemässen Einsetzen des zu applizierenden Insertionskopfes. Der zweite Gehäuseteil kann nach dem Einsetzen des Insertionskopfes in die Haltemittel zurück in die Grundposition bewegt werden. Dabei ist die Insertionsvorrichtung derartig ausgebildet, dass der Insertionskopf nach dem Einsetzen in die Haltemittel und dem Zurückbewegen des zweiten Gehäuseteils in die Grundposition mit den Haltemitteln applikationsbereit, d.h. in einstechbereitem Zustand, in der ersten Position gehalten wird. Bei Insertionsköpfen mit ausklappbaren Kanülen oder Einstechspitzen bedeutet "applikationsbereit", dass die Kanüle oder Einstechspitze vollständig ausgeklappt und falls vorgesehen auch in dieser Position verriegelt ist. Zudem ist die Insertionsvorrichtung derartig ausgebildet, dass der Insertionskopf zu Beginn der Insertionsbewegung von den Haltemitteln separiert wird, so dass er den grössten Teil der Insertionsbewegung losgelöst von den Haltemitteln vollführen kann, bevorzugterweise im "Freiflug" oder allenfalls durch seitliche Führungen geführt, und nach dem Applizieren keine Verbindung mehr zwischen dem Insertionskopf und der Insertionsvorrichtung vorliegt, so dass die Insertionsvorrichtung von dem am Körper applizierten Insertionskopf entfernt werden kann, ohne die Gefahr einer Irritation der Applikationsstelle.

Durch die Erfindung wird die Bereitstellung von Insertionsvorrichtungen für Insertionsköpfe ermöglicht, welche eine sehr kurze Einstechphase mit einem entsprechend kurzen Einstechschmerz ermöglichen, eine Irritation der Einstichstelle beim Lösen des Insertionskopfes von der Insertionsvorrichtung verhindern und gleichzeitig wesentlich einfacher in der Handhabung sind als die heute bekannten gattungsgemässen Insertionsvorrichtungen.

In einer bevorzugten Ausführungsform sind die Haltemittel derartig ausgebildet, dass der Insertionskopf durch diese in der ersten Position rein kraftschlüssig, z.B. durch Klemmung zwischen zwei Federspangen, rein formschlüssig oder kraft- und formschlüssig gehalten werden kann. Insbesondere bei reinem Kraftschluss ergibt sich der Vorteil, dass der Insertionskopf zu Beginn der Insertionsbewegung auf einfache Weise durch ein Vortriebselement der Antriebsmittel aus den Haltemitteln geschlagen werden kann und so von diesen gelöst wird.

Dabei ist es weiter bevorzugt, dass die Insertionsvorrichtung derartig ausgebildet ist, dass der Insertionskopf in der Ladeposition rein formschlüssig oder kraft- und formschlüssig von den Haltemitteln gehalten werden kann, wobei es bevorzugt ist, dass die Insertionsvorrichtung derartig ausgebildet ist, dass der Formschluss beim Bewegen des zweiten Gehäuseteils von der Ladeposition in die Grundposition aufgehoben wird.

Alternativ ist es dabei weiter bevorzugt, dass die Insertionsvorrichtung derartig ausgebildet ist, dass der Insertionskopf in der Ladeposition rein kraftschlüssig von den Haltemitteln gehalten werden kann, wobei es bevorzugt ist, dass die Insertionsvorrichtung derartig ausgebildet ist, dass der Kraftschluss beim Bewegen des zweiten Gehäuseteils von der Ladeposition in die Grundposition verringert wird. Dies kann beispielsweise dadurch erreicht werden, dass der Insertionskopf kraftschlüssig mit Federlaschen gehalten wird, deren freie federnde Länge beim Bewegen von der Ladeposition in die Grundposition verlängert wird. Ebenso sind aber auch Ausführungsvarianten vorgesehen, bei denen sowohl in der Ladeposition als auch in der Grundposition ein reiner Kraftschluss gleicher Stärke vorliegt.

Insbesondere bei der Verwendung von Insertionsköpfen mit feststehender Infusionskanüle oder Einstechspitze ergibt sich bei den Ausführungsvarianten, bei denen bei der Bewegung von der Ladeposition in die Grundposition ein Formschluss aufgehoben oder ein Kraftschluss verringert wird, der Vorteil, dass der Insertionskopf in der Ladeposition fester gehalten wird als in der Grundposition, so dass ein Entfernen des Nadel- bzw. Kanülenschutzes möglich ist ohne Gefahr zu laufen, dabei den Insertionskopf wieder aus den Haltemitteln zu lösen.

In einer weiteren bevorzugten Ausführungsform ist die Insertionsvorrichtung derartig ausgebildet, dass die Haltemittel zumindest während eines Grossteils der Insertionsbewegung oder während der gesamten Insertionsbewegung des Insertionskopfes unbewegt gegenüber der Kontaktfläche sind.

Dabei ist es bevorzugt, wenn die Haltemittel unbeweglich gegenüber der Kontaktfläche bzw. dem Teil der Kontaktflächen, welche bzw. welcher vom ersten Gehäuseteil getragen wird, ausgebildet sind.

In noch einer weiteren bevorzugten Ausführungsform weisen die Antriebsmittel der Insertionsvorrichtung ein oder mehrere Kraftspeicherelemente zur Bereitstellung der Antriebsenergie für die Insertionsbewegung auf, wie z.B. Spiral-, Schenkel- oder Blattfedern aus Metall oder Kunststoff, Gasdruckfedern oder Gummifederelemente. Hierdurch ergibt sich der Vorteil, dass die Insertionsvorrichtung unabhängig von etwaigen Fremdenergiequellen jederzeit und überall einsatzfähig ist und zudem besonders kostengünstig hergestellt werden kann.

Dabei ist es bevorzugt, dass das Kraftspeicherelement entweder durch das Bewegen des zweiten Gehäuseteils von der Grundposition in die Ladeposition oder durch das Bewegen des zweiten Gehäuseteils von der Ladeposition in die Grundposition oder durch beide Bewegungen des zweiten Gehäuseteils vorspannbar ist. Bei der ersten Variante ergibt sich der Vorteil, dass die Kraft zum Zurückbewegen des zweiten Gehäuseteils in die Grundposition relativ gering ausfallen kann, was insbesondere bei der Verwendung von Insertionsköpfen mit feststehenden Infusionskanülen oder Einstechspitzen die Gefahr einer Verletzung des Benutzers durch eine unkontrollierte Bewegung bei eingesetztem Insertionskopf reduziert. Auch ist es hier vorteilhaft, dass die Bewegung, welche die grösste Kraft erfordert, zumindest bei Ausführungen mit schwenkbaren und quer zur Richtung der Insertionsbewegung verschiebbaren zweiten Gehäuseteilen in Zugrichtung der Bewegung erfolgen kann, was gegenüber einer Bewegung in Druckrichtung eine kontrollierte Handhabung der Insertionsvorrichtung begünstigt. Die zweite Variante weist indes den Vorteil auf, dass ein Zusammendrücken von Bauelementen oft als einfacher empfunden wird als ein Auseinanderziehen, so dass diese Variante je nach Ausführung die Bedienung der erfindungsgemässen Insertionsvorrichtung erleichtern kann. Die dritte Variante weist hingegen den Vorteil auf, dass zum Vorspannen des Kraftspeicherelements der doppelte Bewegungsweg zur Verfügung steht, so dass die hierfür erforderliche Kraft deutlich geringer ausfallen kann, was ebenfalls einer kontrollierten Bedienung zuträglich ist. All diese Varianten weisen zudem den Vorteil auf, dass sie die Bereitstellung von kostengünstigen, rein mechanischen Insertionsvorrichtungen ermöglichen, welche jederzeit ohne Hilfsenergie einsatzbereit sind. Dies im Gegensatz zu ebenfalls vorgesehenen bevorzugten Ausführungsformen der erfindungsgemässen Insertionsvorrichtung, bei denen das Vorspannen des Kraftspeicherelements mittels elektrischer Elemente, wie z.B. Elektromotoren, erfolgt. Bei solchen Ausführungsformen können Schalter und/oder Sensoren vorhanden sein, welche die elektrischen Elemente zum Vorspannen der Kraftspeicherelemente ansteuern.

Auch ist es bei diesen Ausführungsvarianten bevorzugt, dass die Insertionsvorrichtung derartig ausgestaltet ist, dass das Kraftspeicherelement bei in der ersten Position befindlichem Insertionskopf im vorgespannten Zustand bereitstellbar ist und sodann die Insertionsbewegung durch Betätigen eines Betätigungsorgans oder durch ein gleichzeitiges oder aufeinander folgendes Betätigen mehrerer Betätigungsorgane auslösbar ist, unter zunehmender Entspannung des Kraftspeicherelements.

Geeignete Betätigungsorgane können rein mechanisch ausgebildet sein, z.B. als Auslöseriegel oder Auslöseschieber, oder z.B. auch elektrische bzw. elektronische Elemente umfassen, z.B. einen elektrisch betätigten Riegel, welcher über einen Schalter und/oder einen Sensor mit Auswerteelektronik gelöst werden kann.

Hierdurch wird eine kontrollierte Applikation des Insertionskopfes begünstigt.

Bei letztgenannter Ausführungsform ist es zudem vorteilhaft, dass die Antriebsmittel ein Vortriebselement zur Übertragung der Antriebskraft auf den zu applizierenden Insertionskopf umfassen und derartig ausgestaltet sind, dass das Kraftspeicherelement unter einem Verschieben des Vortriebselements entgegen der Richtung der Insertionsbewegung und unter anschliessendem Verrasten desselben mit durch die Betätigungsorgane lösbaren Rastmitteln vorspannbar und in vorgespanntem Zustand bereitstellbar ist. Solche Konstruktionen lassen sich kostengünstig realisieren, sind funktionssicher und ermöglichen zudem eine hohe Anfangsbeschleunigung und damit eine schnelle Insertionsbewegung, wodurch sich der Applikationsschmerz beim Einstechen der Infusionskanüle oder der Einstechspitze in den Körper minimieren lässt. Erfindungsgemäß ist der zweite Gehäuseteil zum Bewegen von der Grundposition in die Ladeposition und zurück gegenüber dem ersten Gehäuseteil schwenkbar, was eine einfache konstruktive Ausgestaltung der Verbindung beider Gehäuseteile miteinander begünstigt.

In einer alternativen erfindungsgemäßen Ausführungsform ist der zweite Gehäuseteil zum Bewegen von der Grundposition in die Ladeposition und zurück gegenüber dem ersten Gehäuseteil verschiebbar, bevorzugterweise quer, noch bevorzugter senkrecht zur Richtung der Insertionsbewegung. Hierdurch eröffnen sich interessante Alternativen gegenüber der zuvor beschriebenen Ausführungsform hinsichtlich der konstruktiven Ausgestaltung der Verbindung der beiden Gehäuseteile.

In noch einer weiteren bevorzugten Ausführungsform der Insertionsvorrichtung trägt der zweite Gehäuseteil einen Teil der Kontaktflächen. Entsprechend wird dieser Teil der Kontaktflächen beim Bewegen des zweiten Gehäuseteils von der Grundposition in die Ladeposition vorübergehend von den übrigen, vom ersten Gehäuseteil getragenen Kontaktflächen entfernt, wodurch Ausführungen möglich werden, bei denen die Haltemittel in der Ladeposition besonders gut zugänglich sind.

In noch einer weiteren bevorzugten Ausführungsform sind die Haltemittel derartig ausgebildet, dass der Insertionskopf durch ein Einschieben in einer Richtung quer, insbesondere senkrecht zur Richtung der Insertionsbewegung in diese eingesetzt werden kann. Hierdurch kann insbesondere bei Verwendung von Insertionsköpfen mit feststehenden Kanülen oder Einstechspitzen die Verletzungsgefahr deutlich herabgesetzt werden.

In noch einer weiteren bevorzugten Ausführungsform weist die Insertionsvorrichtung mindestens zwei Betätigungsorgane auf, welche zur Auslösung der Insertionsbewegung gleichzeitig betätigt sein müssen. Dabei ist ein erstes der Betätigungsorgane bevorzugterweise derartig ausgebildet, dass es durch ein Andrücken der Insertionsvorrichtung mit der Kontaktfläche an den Körper des Patienten betätigt werden kann, und zwar bevorzugterweise durch ein Andrücken derselben in Richtung der Insertionsbewegung, was insbesondere dann sinnvoll ist, wenn die Insertion unter einem Winkel von etwa 90° zur Körperoberfläche erfolgt. Durch diese Ausgestaltung der Insertionsvorrichtung wird die Gefahr eines versehentlichen Auslösens der applikationsbereiten Insertionsvorrichtung und damit die Wahrscheinlichkeit einer Verletzung des Bedieners deutlich herabgesetzt.

Bevorzugterweise ist dabei das erste Betätigungsorgan als schieber- oder tasterförmiges Element ausgebildet, wobei es weiter bevorzugt ist, wenn dieses gleichzeitig die Kontaktfläche oder aber, bei mehreren Kontaktflächen, sämtliche Kontaktflächen der Insertionsvorrichtung bildet. Hierdurch ergibt sich der Vorteil, dass eine sichere Betätigung des ersten Betätigungsorgans unabhängig von der Oberflächenkontur der Applikationsstelle am Körper des Patienten möglich ist.

Alternativ zu rein mechanischen Ausführungsformen des ersten Betätigungsorgans ist es auch bevorzugt, elektrische erste Betätigungsorgane vorzusehen. Dies ist beispielsweise möglich in Form eines elektrischen Riegelelements, welches mittels eines oder mehrerer an der Kontaktfläche angeordneter Hautkontaktsensoren (z.B. Leitfähigkeitssensoren) und einer zugeordneten Steuerelektronik angesteuert wird, derart, dass dieses beim bestimmungsgemässen Aufsetzen der Insertionsvorrichtung auf die Applikationsstelle aktiviert wird.

Zudem ist es bei den Ausführungsformen mit mindestens zwei Betätigungsorganen bevorzugt, dass zumindest eines der Betätigungsorgane, anspruchsgemäss als zweites Betätigungsorgan bezeichnet, als tasten- oder knopfförmiges Element ausgebildet ist, welches durch Drücken mit einer Fingerkuppe des Bedieners betätigt werden kann. Dabei ist die Betätigungsrichtung bevorzugterweise quer, insbesondere senkrecht zur Andrückrichtung der Insertionsvorrichtung an den Körper des Patienten, welche bevorzugterweise gleich der Richtung der anspruchsgemässen Insertionsbewegung und damit der Einstichrichtung der Infusionskanüle oder der Einstechspitze ist. Dieses zweite Betätigungsorgan kann rein mechanisch ausgebildet sein, z.B. als Auslöseriegel oder Auslöseschieber, oder z.B. auch elektrische bzw. elektronische Elemente umfassen, z.B. einen elektrisch betätigten Riegel, welcher über einen Schalter und/oder einen Sensor mit Auswerteelektronik gelöst werden kann.

Durch diese Ausgestaltung lässt sich die Gefahr einer unbeabsichtigten Betätigung dieses Betätigungsorgans zusammen mit dem anspruchsgemässen ersten Betätigungsorgan weiter herabsetzten, so dass die Gefahr einer unbeabsichtigten Auslösung der Insertionsvorrichtung weiter gesenkt wird.

Bei bestimmten Ausführungsformen kann es auch vorteilhaft sein, wenn die Betätigungsrichtung parallel oder im wesentlichen parallel zur Andrückrichtung ist.

In noch einer weiteren bevorzugten Ausführungsform der erfindungsgemässen Insertionsvorrichtung mit mindestens zwei Betätigungsorganen sind deren für die Auslösung der Insertionsbewegung zu betätigenden Betätigungsorgane derartig miteinander gekoppelt, dass durch die Betätigung eines dieser Betätigungsorgane eine Sperrung eines anderen oder mehrerer anderer dieser Betätigungsorgane aufhebbar ist. Hierdurch kann die Konstruktion vereinfacht werden, da nur ein einziger Auslösemechanismus erforderlich ist. Insbesondere bei Vorhandensein eines Betätigungsorgans mit elektrischen Elementen kann hierdurch ein besonders einfacher kostengünstiger Aufbau realisiert werden, z.B. indem ein elektrisches Riegelelement nur dann auslösbar ist, wenn zwei Schalter oder Sensoren gleichzeitig betätigt werden oder eine Kombination aus mindestens einem Schalter und mindestens einem Sensor.

In noch einer weiteren bevorzugten Ausführungsform der Insertionsvorrichtung mit einem oder mindestens zwei Betätigungsorganen sind sämtliche Betätigungsorgane, welche zur Auslösung der Insertionsbewegung gleichzeitig betätigt sein müssen, derartig ausgebildet, dass sie vom Bediener mit einer Hand betätigt werden können. Entsprechend ist eine einhändige Bedienung der Insertionsvorrichtung möglich, wodurch die Applikation des Insertionskopfes durch den Patienten selbst an für diesen beidhändig nicht oder nur sehr schwer zugänglichen Körperstellen, wie z.B. im Bereich der Hüfte, möglich wird.

In noch einer weiteren bevorzugten Ausführungsform der Insertionsvorrichtung mit einem oder mehreren Betätigungsorganen sind die Betätigungsorgane, welche zur Auslösung der Insertionsbewegung gleichzeitig betätigt sein müssen, derartig ausgebildet, dass diese bei Wegfall einer Betätigungskraft automatisch wieder ihren unbetätigten Zustand annehmen. Hierdurch lässt sich die Sicherheit gegen ein unbeabsichtigte Auslösen der Insertionsvorrichtung weiter steigern.

In noch einer bevorzugten Ausführungsform weist die Insertionsvorrichtung Mittel zur Bewirkung einer Verschiebung, insbesondere einer Querverschiebung, eines verschieblichen Aktivierungsorgans eines in den Haltemitteln aufzunehmenden Insertionskopfes mit einer einzigen oder mehreren ausklappbaren Infusionskanülen und/oder einer einzigen oder mehreren ausklappbaren Einstechspitzen während dem Bewegen des zweiten Gehäuseteils von der Ladeposition in die Grundposition auf, zur Ermöglichung eines automatischen Ausklappens sämtlicher ausklappbarer Infusionskanülen und sämtlicher ausklappbarer Einstechspitzen des Insertionskopfes während dem Bewegen des zweiten Gehäuseteils von der Ladeposition in die Grundposition. Hierdurch wird es möglich, auf die Insertionsvorrichtung angepasste Insertionsköpfe mit ausklappbaren Infusionskanülen bzw. Einstechspitzen im Schutzzustand, d.h. mit eingeklappten Kanülen bzw. Einstechspitzen, in der Ladeposition in die Haltemittel der Insertionsvorrichtung einzusetzen und die Kanüle bzw. Einstechspitze des Insertionskopf sodann automatisch durch Bewegen des zweiten Gehäuseteils in die Grundposition auszuklappen, wodurch der Insertionskopf in den applikationsbereiten Zustand gebracht wird. Hierdurch kann die Gefahr einer Verletzung des Bedieners mit den Kanülen oder den Einstechspitzen bei der Vorbereitung der Applikation des Insertionskopfes praktisch eliminiert werden.

Bei der zuvor beschriebenen Ausführungsform ist es bevorzugt, dass die Mittel zur Bewirkung einer Verschiebung eines verschieblichen Aktivierungsorgans eine vom zweiten Gehäuseteil gebildete Anlauffläche umfassen, mittels welcher ein bevorzugterweise quer zur Richtung der Insertionsbewegung verschiebliches Aktivierungsorgan eines entsprechend ausgebildeten und in den Haltemitteln zu haltenden Insertionskopfes mit ausklappbarer Infusionskanüle oder Einstechspitze während dem Bewegen des zweiten Gehäuseteils von der Ladeposition in die Grundposition verschoben werden kann, zur Bewirkung eines automatischen Ausklappens der ausklappbaren Infusionskanülen bzw. Einstechspitzen des Insertionskopfes während dem Bewegen des zweiten Gehäuseteils von der Ladeposition in die Grundposition. Solche Mittel zur Bewirkung einer Verschiebung eines verschieblichen Aktivierungsorgans lassen sich denkbar einfach und kostengünstig zur Verfügung stellen.

Bei den beiden zuvor beschriebenen Ausführungsformen ist es auch bevorzugt, dass die Mittel zur Bewirkung einer Verschiebung eines verschieblichen Aktivierungsorgans einen Hebelmechanismus umfassen, mit welchem ein bevorzugterweise quer zur Richtung der Insertionsbewegung verschiebliches Aktivierungsorgan eines entsprechend ausgebildeten und in den Haltemitteln zu haltenden Insertionskopfes mit ausklappbarer Infusionskanüle oder Einstechspitze während dem Bewegen des zweiten Gehäuseteils von der Ladeposition in die Grundposition verschoben werden kann, zur Bewirkung eines automatischen Ausklappens der ausklappbaren Infusionskanülen bzw. Einstechspitzen des Insertionskopfes während dem Bewegen des zweiten Gehäuseteils von der Ladeposition in die Grundposition. Hierdurch ergeben sich grosse Freiräume für die konstruktive Ausgestaltung sowohl der Insertionsvorrichtung als auch des zugehörigen Insertionskopfes.

Auch ist es von Vorteil, wenn die Insertionsvorrichtung derartig ausgestaltet ist, dass sie mehrfach zum Applizieren eines Insertionskopfes verwendet werden kann. Hierdurch lassen sich Kosten sparen.

Ein zweiter Aspekt der Erfindung betrifft eine Anordnung, welche eine Insertionsvorrichtung gemäss dem ersten Aspekt der Erfindung und einen in dieser aufgenommenen oder aufnehmbaren, bevorzugterweise als Infusionsset, Port und/oder Sensoranordnung ausgebildeten Insertionskopf mit mindestens einer Infusionskanüle und/- oder mindestens einer Einstechspitze umfasst. Der Insertionskopf kann also z.B. eine einzelne Infusionskanüle oder Einstechspitze aufweisen, mehrere Kanülen oder Einstechspitzen aufweisen oder auch eine oder mehrere Kanülen und eine oder mehrere Einstechspitzen umfassen. Durch Einsetzen eines entsprechenden passenden, zu applizierenden Insertionskopfes in die erfindungsgemässe Insertionsvorrichtung gemäss dem ersten Aspekt der Erfindung entsteht zwangsläufig eine solche erfindungsgemässe Anordnung. Dabei ist es neben der Vermarktung von mehrfach verwendbaren erfindungsgemässen Insertionsvorrichtungen gemäss dem ersten Aspekt der Erfindung und zugehörigen Insertionsköpfen, wie zugehörigen Infusionssets, Ports oder Sensoranordnungen, welche vom Bediener kurz vor der Verwendung zu erfindungsgemässen Anordnungen zusammengefügt werden, auch vorgesehen, bereits fertig zusammengestellte erfindungsgemässe Anordnungen als Einwegartikel anzubieten, bei denen die Insertionsvorrichtung nach dem Applizieren des Insertionskopfes entsorgt wird.

In einer bevorzugten Ausführungsform der erfindungsgemässen Anordnung ist der Insertionskopf ein Insertionskopf mit mindestens einer ausklappbaren Infusionskanüle und/oder mindestens einer Einstechspitze, wobei sämtliche Infusionskanülen und Einstechspitzen des Insertionskopfes im nicht-aktivierten Zustand, in welchem der Insertionskopf zur Aufnahme in der Ladeposition in den Haltemittel vorgesehen ist oder bereits in den Haltemitteln aufgenommen ist, eingeklappt sind und die Insertionsvorrichtung und der Insertionskopf derartig ausgebildet sind, dass sämtliche ausklappbaren Infusionskanülen und Einstechspitzen des Insertionskopfes beim Bewegen des zweiten Gehäuseteils von der Ladeposition in die Grundposition automatisch ausgeklappt werden. Wie bereits anlässlich der Diskussion des ersten Aspekts der Erfindung erwähnt, kann hierdurch die Gefahr einer Verletzung des Bedieners an einer Kanüle oder Einstechspitze bei der Vorbereitung der Applikation des Insertionskopfes praktisch ausgeschlossen werden.

Bevorzugt ist die Verwendung der Insertionsvorrichtung oder der Anordnung nach einem der vorangehenden Aspekte der Erfindung zum Applizieren eines bevorzugterweise als Infusionsset, Port und/oder Sensoranordnung ausgebildeten Insertionskopfes mit mindestens einer Infusionskanüle und/oder mindestens einer Einstechspitze am Körper eines Patienten. Solche Verwendungen sind bestimmungsgemäss und lassen die Vorteile der Erfindung besonders deutlich zu Tage treten.

Im Folgenden wird ein Verfahren zum Applizieren eines bevorzugterweise als Infusionsset, Port und/oder Sensoranordnung ausgebildeten Insertionskopfes am Körper eines Patienten mit einer Insertionsvorrichtung gemäss dem ersten Aspekt der Erfindung beschrieben.

Dabei wird in einem ersten Verfahrensschritt die Insertionsvorrichtung gemäss dem ersten Aspekt der Erfindung mit dem zweiten Gehäuseteil angeordnet in der Grundposition bereitgestellt, in welcher ein Einsetzen des Insertionskopfes in die Haltemittel nicht oder nur schwierig möglich ist.

Sodann wird in einem zweiten Schritt das zweite Gehäuseteil von der Grundposition in die Ladeposition bewegt, wodurch die Haltemittel zum Einsetzen des Insertionskopfes zugänglich gemacht werden.

In einem dritten Schritt wird der auf die Insertionsvorrichtung angepasste Insertionskopf mit mindestens einer Infusionskanüle und/oder mindestens einer Einstechspitze in die Haltemittel eingesetzt, derart, dass dieser von den Haltemitteln gehalten wird. Es liegt also eine erfindungsgemässe Anordnung gemäss dem zweiten Aspekt vor, wobei der Insertionskopf eine einzelne Infusionskanüle oder Einstechspitze, mehrere Kanülen oder Einstechspitzen oder auch eine oder mehrere Kanülen und eine oder mehrere Einstechspitzen aufweisen kann.

Sodann wird in einem vierten Schritt das zweite Gehäuseteil ausgehend von der Ladeposition in die Grundposition zurückbewegt, wobei der Insertionskopf nach dem Erreichen der Grundposition durch die Haltemittel applikationsbereit in der ersten Position gehalten wird. Die Insertionsvorrichtung ist nun bereit für den eigentlichen Applikationsvorgang.

Hierzu wird die Insertionsvorrichtung in einem fünften Schritt des Verfahrens mit der oder den Kontaktflächen der Insertionsvorrichtung an der gewünschten Applikationsstelle am Körper des Patienten derartig angeordnet, dass die Infusionskanülen bzw. Einstechspitzen des Insertionskopfes bei der anschliessend zu erfolgenden Insertionsbewegung bestimmungsgemäss in den Körper eindringen können.

Sodann wird in einem sechsten Schritt die Insertionsbewegung des Insertionskopfes ausgelöst, wobei der Insertionskopf zu Beginn der Insertionsbewegung von den Haltemitteln separiert wird, derart, dass dieser zumindest den grössten Teil der Insertionsbewegung losgelöst von den Haltemitteln vollführt.

Durch dieses Verfahren zum Applizieren eines Insertionskopfes mit einer Insertionsvorrichtung am Körper eines Patienten wird eine kurze Einstechphase mit einem entsprechend kurzen Einstechschmerz ermöglicht und eine Irritation der Einstichstelle beim Lösen des Insertionskopfes von der Insertionsvorrichtung kann sicher verhindert werden, bei gleichzeitiger Vereinfachung der Verfahrensdurchführung gegenüber heute bekannten gattungsgemässen Verfahren.

In einer bevorzugten Ausführungsform des Verfahrens wird der Insertionskopf in der ersten Position rein kraftschlüssig, rein formschlüssig oder kraft- und formschlüssig mit den Haltemitteln gehalten. Hierdurch ergibt sich insbesondere bei reinem Kraftschluss der Vorteil, dass der Insertionskopf zu Beginn der Insertionsbewegung auf einfache Weise durch ein Vortriebselement der Antriebsmittel aus den Haltemittel geschlagen werden kann und so von diesen gelöst wird.

Dabei ist es bevorzugt, wenn der Insertionskopf nach dem Einsetzen in die Haltemittel in der Ladeposition rein formschlüssig oder kraftschlüssig und formschlüssig mit den Haltemitteln gehalten wird, wobei es bevorzugt ist, dass der Formschluss beim Bewegen des zweiten Gehäuseteils von der Ladeposition in die Grundposition aufgehoben wird.

Alternativ ist es dabei bevorzugt, wenn der Insertionskopf nach dem Einsetzen in die Haltemittel in der Ladeposition rein kraftschlüssig mit den Haltemitteln gehalten wird, wobei es bevorzugt ist, dass der Kraftschluss beim Bewegen des zweiten Gehäuseteils von der Ladeposition in die Grundposition verringert wird. Es sind aber auch Ausführungsformen vorgesehen, bei denen der Insertionskopf sowohl in der Ladeposition als auch in der Grundposition rein kraftschlüssig mit einem jeweils gleich starken Kraftschluss gehalten wird.

Insbesondere bei der Verwendung von Insertionsköpfen mit feststehenden Infusionskanülen bzw. Einstechspitzen ergibt sich bei denjenigen Verfahrensvarianten, bei denen bei der Bewegung von der Ladeposition in die Grundposition ein Formschluss aufgehoben bzw. ein Kraftschluss verringert wird, der Vorteil, dass der Insertionskopf in der Ladeposition fester gehalten wird als in der Grundposition, so dass ein Entfernen des Nadel- bzw. Kanülenschutzes möglich ist ohne Gefahr zu laufen, dabei den Insertionskopf wieder aus den Haltemitteln zu lösen.

In noch einer bevorzugten Ausführungsform des Verfahrens kommt eine Insertionsvorrichtung zum Einsatz, deren Antriebsmittel mindestens ein vorspannbares Kraftspeicherelement zur Bereitstellung der Antriebsenergie für die Insertionsbewegung aufweisen, wobei das Kraftspeicherelement durch das Bewegen des zweiten Gehäuseteils von der Grundposition in die Ladeposition und/oder durch das Bewegen des zweiten Gehäuseteils von der Ladeposition in die Grundposition vorgespannt wird. Die einzelnen Verfahrensvarianten weisen verschiedene Vorteile auf, welche bereits bei der Diskussion der entsprechenden Ausführungsformen des ersten Aspekts der Erfindung dargelegt wurden. All diese Varianten weisen ausserdem den Vorteil auf, dass sie die Verwendung von kostengünstigen, rein mechanischen Insertionsvorrichtungen begünstigen, welche jederzeit ohne Hilfsenergie einsatzbereit sind. Dies im Gegensatz zur ebenfalls vorgesehenen Verwendung von Insertionsvorrichtung, bei denen das Vorspannen des Kraftspeicherelements mittels elektrischer Elemente, wie z.B. Elektromotoren, erfolgt.

In einer weiteren bevorzugten Ausführungsform des Verfahrens wird der zweite Gehäuseteil beim Bewegen von der Grundposition in die Ladeposition und umgekehrt gegenüber dem ersten Gehäuseteil geschwenkt, was die Verwendung einfacher und robuster erfindungsgemässer Insertionsvorrichtungen ermöglicht.

In noch einer weiteren bevorzugten Ausführungsform des Verfahrens wird der Insertionskopf in der ersten Position kraftschlüssig in den Haltemitteln gehalten, wobei die Haltemittel während der Insertionsbewegung des Insertionskopfes unbewegt gegenüber der Kontaktfläche gehalten werden, was bevorzugterweise dadurch bewerkstelligt wird, dass diese unbeweglich gegenüber der bzw. den vom ersten Gehäuseteil bereitgestellten Kontaktflächen ausgebildet werden. Hierdurch wird die Verwendung von konstruktiv einfach aufgebauten und entsprechend kostengünstigen und robusten erfindungsgemässen Insertionsvorrichtungen möglich.

In noch einer weiteren bevorzugten Ausführungsform des Verfahrens kommt eine Insertionsvorrichtung zum Einsatz, deren Antriebsmittel mindestens ein vorspannbares Kraftspeicherelement zur Bereitstellung der Antriebsenergie für die Insertionsbewegung aufweisen, wobei das Kraftspeicherelement im vorgespannten Zustand bereitgestellt wird und sodann die Insertionsbewegung durch Betätigen eines Betätigungsorgans oder durch ein gleichzeitiges oder aufeinander folgendes Betätigen mehrerer Betätigungsorgane ausgelöst wird. Dies begünstigt einen kontrollierten Applikationsvorgang.

Auch ist es bevorzugt, dass das Anordnen der Insertionsvorrichtung am Körper des Patienten und das Auslösen der Insertionsbewegung mit einer Hand erfolgt, so dass auch schwierig und nur mit einer Hand erreichbare Körperstellen für die Applikation bereitstehen.

In noch einer weiteren bevorzugten Ausführungsform des Verfahrens wird ein Insertionskopf mit mindestens einer ausklappbaren Infusionskanüle und/oder mindestens einer ausklappbaren Einstechspitze in einem Zustand, in welchem sämtliche ausklappbaren Infusionskanülen und Einstechspitzen des Insertionskopfes eingeklappt sind, in die Haltemittel eingesetzt. Sodann werden sämtliche ausklappbaren Infusionskanülen und Einstechspitzen des Insertionskopfes beim Bewegen des zweiten Gehäuseteils von der Ladeposition in die Grundposition ausgeklappt. Der Insertionskopf ist sodann applikationsbereit. Hierdurch lässt sich die Verletzungsgefahr für den Bediener bei der Vorbereitung der Applikation praktisch auf Null reduzieren.

Weitere bevorzugte Ausführungen der Erfindung ergeben sich aus den abhängigen Ansprüchen und aus der nun folgenden Beschreibung anhand der Figuren. Dabei zeigen:
Fig. 1 einen Vertikalschnitt durch eine erfindungsgemässe Insertionsvorrichtung im nicht vorgespannten Grundzustand ohne Insertionskopf;
Fig. 2 einen Vertikalschnitt durch die Insertionsvorrichtung aus Fig. 1 während dem Vorspannen;
Fig. 3 einen Vertikalschnitt durch die Insertionsvorrichtung aus Fig. 1 am Ende des Vorspannvorgangs;
Fig. 4 eine Darstellung der Insertionsvorrichtung wie Fig. 3, jedoch mit darin angeordnetem Insertionskopf;
Fig. 5 einen Vertikalschnitt durch die applikationsbereite, gesicherte Insertionsvorrichtung;
Fig. 6 eine Darstellung der Insertionsvorrichtung wie Fig. 5, jedoch im entsicherten Zustand; und
Fig. 7 die Insertionsvorrichtung aus Fig. 6 kurz nach dem Betätigen der Auslösetaste.

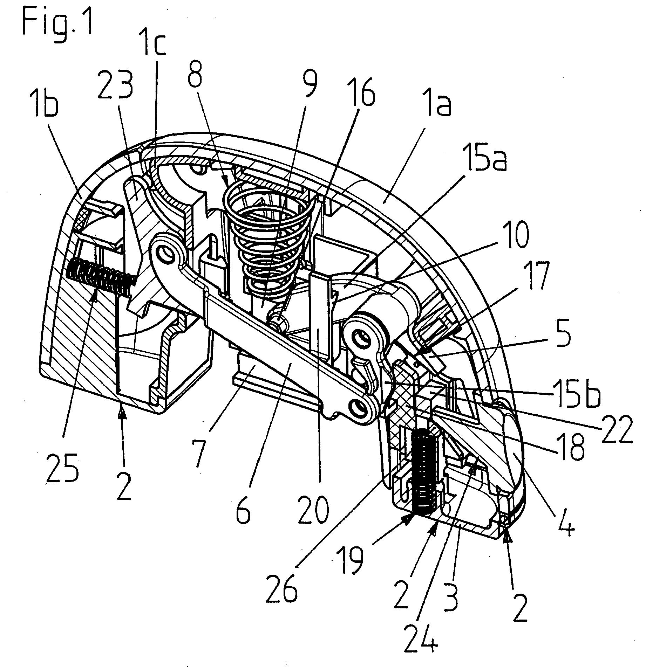

Eine erfindungsgemässe Insertionsvorrichtung für Insertionsköpfe mit ausklappbarer Infusionskanüle ist in einem Vertikalschnitt im nicht vorgespannten Zustand und ohne Insertionskopf in Fig. 1 dargestellt. Wie zu erkennen ist, weist die Insertionsvorrichtung ein portalartiges Gehäuse 1a, 1b auf, welches an seiner Unterseite Kontaktflächen 2 zum Aufsetzen und Andrücken der Insertionsvorrichtung auf den Körper eines Patienten beim Applizieren eines Insertionskopfes, z.B. eines Infusionssets, mit der Insertionsvorrichtung aufweist. Eine der Kontaktflächen 2 wird von einem Sicherungstaster 3 gebildet, welcher an der Unterseite des Gehäuses 1a, 1b nach unten vorsteht und zum Entsichern der Insertionsvorrichtung, wenn sich diese in einem applikationsbereiten Zustand befindet, durch Aufsetzen auf und Andrücken an den Körper des Patienten in eine Entsicherungsposition verschoben werden kann, in welcher die Kontaktfläche 2 des Sicherungstasters 3 im wesentlichen oberflächenbündig mit der an den Sicherungstaster 3 angrenzenden Kontaktfläche 2 des Gehäuses 1a, 1b ist.

Weiter weist die Insertionsvorrichtung einen Auslöseknopf 4 auf, mit welchem bei in einem applikationsbereiten Zustand sich befindlicher Insertionsvorrichtung und bei in der Entsicherungsposition angeordnetem Sicherungstaster 3 die Insertionsbewegung zum Applizieren des Insertionskopfes am Körper des Patienten ausgelöst werden kann. Sicherungstaster 3 und Auslöseknopf 4 stellen also zwei anspruchsgemässe Betätigungsorgane 3, 4 dar, welche zur Auslösung der Insertionsbewegung gleichzeitig betätigt sein müssen.

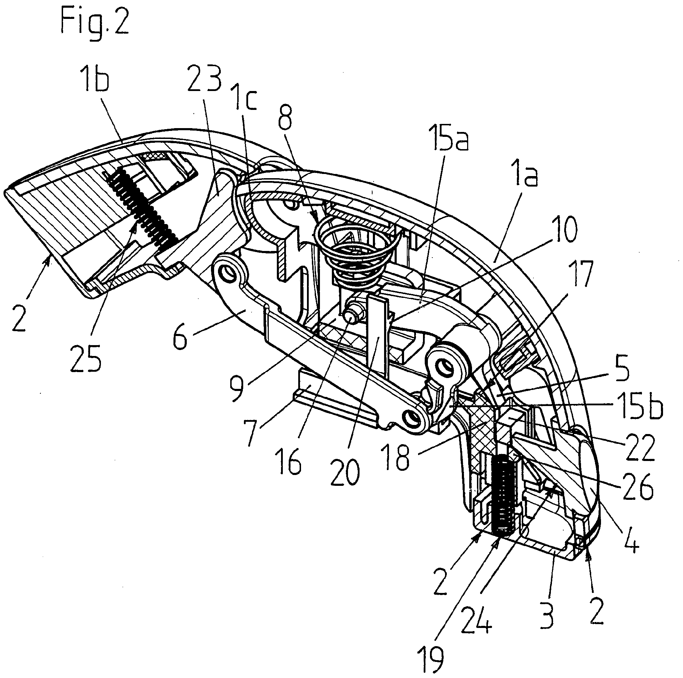

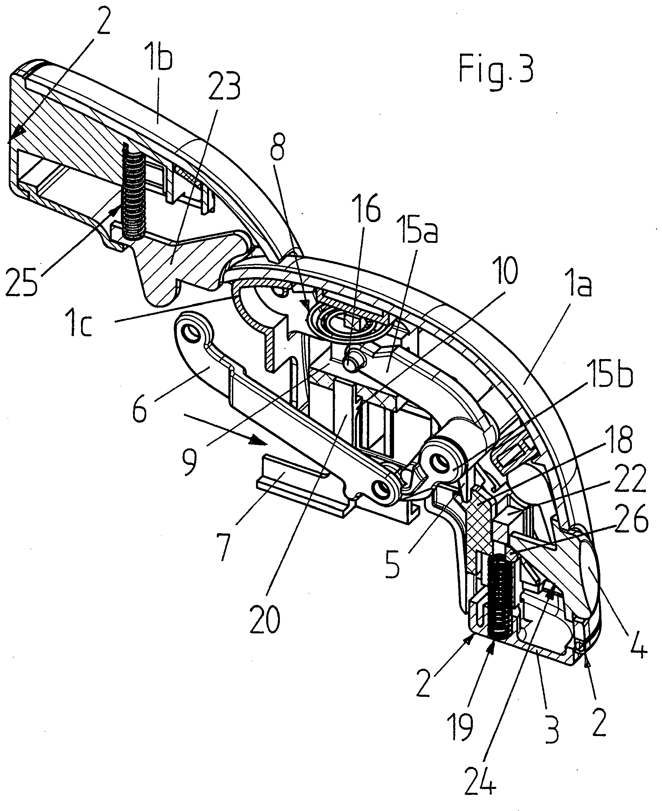

Wie in Zusammenschau mit den Figuren 2 und 3 erkennbar ist, welche die Insertionsvorrichtung einmal während dem Vorspannen (Fig. 2) und einmal am Ende des Vorspannvorgangs (Fig. 3) zeigen, weist das Gehäuse 1a, 1b einen ersten Gehäuseteil 1a auf, in welchem ein fest mit diesem verbundener innerer Gehäuseteil 1c angeordnet ist, welcher als anspruchsgemässe Haltemittel zwei sich gegenüberliegende federlaschenartige Halteelemente (nicht sichtbar) zum kraftschlüssigen Halten eines mit der Insertionsvorrichtung zu applizierenden Insertionskopfes trägt, und einen zweiten Gehäuseteil 1b, welcher gegenüber dem ersten Gehäuseteil 1a von der in Fig. 1 dargestellten Grundposition in die in Fig. 3 dargestellte Ladeposition geschwenkt werden kann.

Wie weiter zu erkennen ist, umfasst die Insertionsvorrichtung als Antriebsmittel eine Spiralfeder 8, die auf ein Hammerelement 9 wirkt. Spiralfeder 8 und Hammerelement 9 sind innerhalb des ersten Gehäuseteils 1a angeordnet.

Das Hammerelement 9 ist über einen als Doppelhebel ausgebildeten und schwenkbar im ersten Gehäuseteil 1a gelagerten Auslöserhebel 15a, 15b und eine Zuglasche 6 mit dem zweiten Gehäuseteil 1b verbunden, derart, dass beim Nachaussenschwenken des zweiten Gehäuseteils 1b das Hammerelement 9 entgegen der Kraft der Spiralfeder 8 im ersten Gehäuseteil 1a nach oben bewegt wird, unter zunehmender Vorspannung der Spiralfeder 8. Hierzu weist der Auslöserhebel 15a, 15b an seinem hammerelementseitigen Ende zwei Führungszylinder 16 (nur einer gezeigt) auf, die, einem Gleitstein vergleichbar, in sich gegenüberliegenden horizontalen Langlöchern (nicht gezeigt) im Hammerelement 9 geführt sind. Das andere Ende des Auslöserhebels 15 ist mit einem Ende der Zuglasche 6 verbunden, deren anderes Ende wiederum mit dem zweiten Gehäuseteil 1b verbunden ist, allerdings im Bereich eines für die Schnittdarstellung weggeschnittenen Bereichs, weshalb diese Anbindung in den Figuren nicht erkennbar ist.

Wie weiter zu erkennen ist, weist der Auslöserhebel 15a, 15b einen Rastvorsprung 5 mit einer Anlauframpe 17 auf, mit welcher er bei seiner das zunehmende Vorspannen der Feder 8 bewirkenden Schwenkbewegung ein Riegelelement 18 entgegen der Kraft einer Verriegelungsfeder 19 nach unten bewegt, bis der Rastvorsprung 5 über das Riegelelement 18 hinaus läuft und sodann durch das unter der Kraft der Verriegelungsfeder 19 zurückschnellende Riegelelement 18 in der in Fig. 3 dargestellten Situation verriegelt wird.

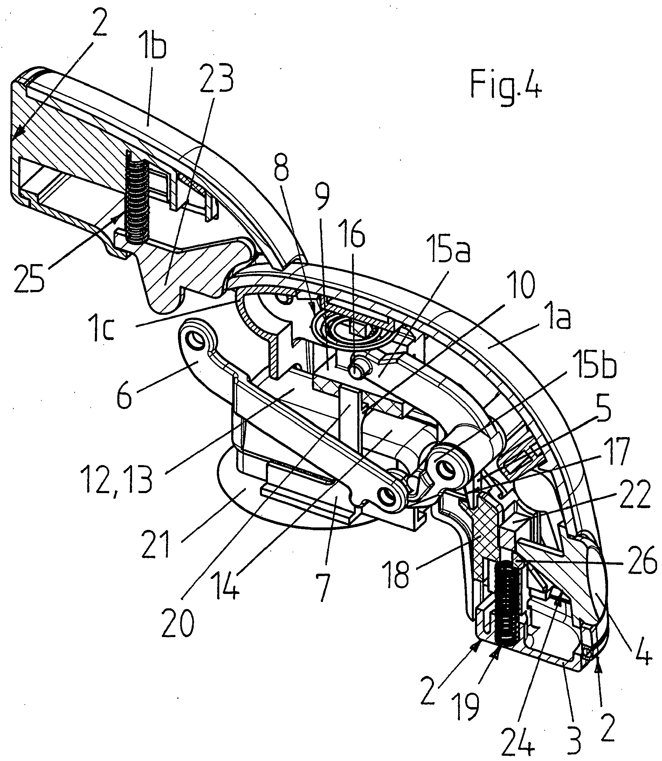

Bei Erreichen der oberen, in Fig. 3 dargestellten maximal vorgespannten Position verrastet das Hammerelement 9 mit einer Rastnase 10, welche an einem Federarm 20 eines im ersten Gehäuseteil 1a entgegen der Kraft einer (nicht gezeigten) Rückstellfeder horizontal verschiebbaren Schiebers 7 gebildet ist, so dass ein Auslösen des Hammerelements 9 schon allein durch diese Rastnase 10 verunmöglicht ist. In diesem Zustand, in welchem das zweite Gehäuseteil 1b maximal gegenüber dem ersten Gehäuseteil 1a verschwenkt ist und welcher dem anspruchsgemässen Ladezustand entspricht, kann nun ein passender Insertionskopf 12 in der in Fig. 3 durch einen Pfeil angedeuteten Richtung zwischen die Halteelementen geschoben und dadurch an diesen gehalten werden, wie dies in Fig. 4 gezeigt ist.

Um die Insertionsvorrichtung in einen applikationsbereiten Zustand zu versetzen, muss das zweite Gehäuseteil nun von der in Fig. 4 dargestellten Ladeposition zurück in die Grundposition geschwenkt werden, was trotz verriegeltem Auslöserhebel 15a möglich ist, da der mit der Zuglasche 6 verbundene Arm 15b des Auslöserhebels gegenüber dem mit dem Hammerelement 6 und dem Rastvorsprung 5 verbunden Arm 15a desselben drehbar um einen entsprechenden Drehwinkel verbunden ist. In diesem vorgespannten Grundzustand verrastet das zweite Gehäuseteil 1b reversibel mit dem ersten Gehäuseteil 1a mittels (nicht dargestellter) Rastmittel, derart, dass es nur nach Überwindung eines erhöhten Anfangswiderstandes in die entgegen gesetzte Richtung und wieder in die Ladeposition bewegt werden kann. Geeignete Rastmittel sind dem Fachmann bekannt und könnten beispielsweise durch eine an eine Federzunge gehaltene Rastnase gebildet werden, welche mit einer Anlaufschräge in einen Hinterschnitt einrastet, so dass die Rastverbindung unter einem mit erhöhtem Kraftaufwand einhergehenden Auslenken der Federzunge durch Anlaufen der Anlaufschräge an einer Kante des Hinterschnitts wieder gelöst werden kann. Alternativ ist es auch vorgesehen, die Rastmittel derart auszubilden, .dass die Verrastung durch Betätigung von zugeordneten Entriegelungsmitteln, wie z.B. durch Drücken einer Entriegelungstaste, wieder gelöst werden kann.

Gleichzeitig mit der Zurückschwenkbewegung des zweiten Gehäuseteils 1b in die Grundposition wird die Kanüle 11 des Insertionskopfes 12 ausgeklappt, indem ein schwenkbar entgegen der Kraft einer Stützfeder 25 am zweiten Gehäuseteil 1b gelagerter Aktivierungshebel 23 mit dem zweiten Gehäuseteil 1b auf den Insertionskopf 12 zu geschwenkt wird, wobei er den linken Gehäuseteil 13 des Insertionskopfes 12, welcher ein verschiebliches Aktivierungsorgan des Insertionskopfes 12 bildet, in den Halteelementen horizontal verschiebt und dabei über den rechten Gehäuseteil 14 desselben, welcher sich im ersten Gehäuseteil 1a der Insertionsvorrichtung abstützt, schiebt und dadurch über einen insertionskopfinternen Mechanismus (nicht gezeigt) die Kanüle 11 ausklappt. Im vorliegenden Fall ist der Insertionskopf 12 ein Infusionsset für Insulin, welches im hier gezeigten Zustand vor der Applikation eine von einer nach der Applikation zu entfernenden Einstechnadel gestützte flexible Kanüle (Softkanüle) aufweist und an seiner Unterseite ein Befestigungspflaster 21 trägt. Für die Zwecke der Darlegung der Erfindung muss hier jedoch nicht zwischen Einstechnadel und flexibler Kanüle unterschieden werden, weshalb beide zusammen in diesem Beispiel als "Kanüle 11" bezeichnet werden.

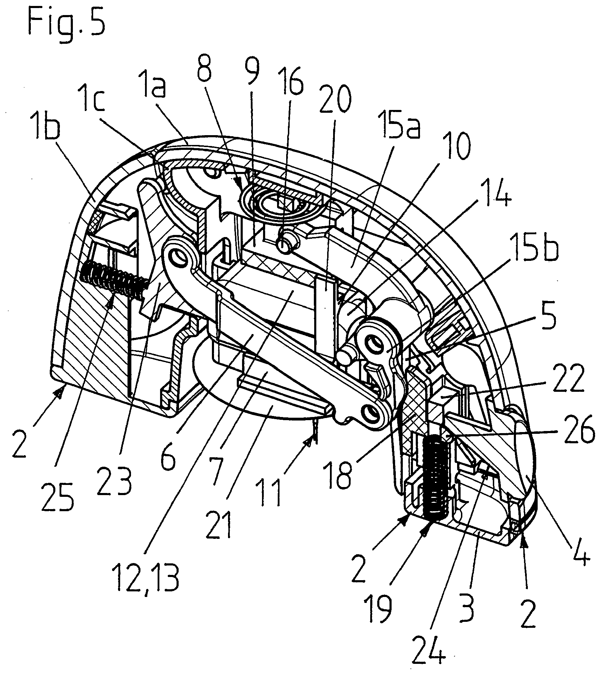

Bei seiner Verschiebung nimmt der linke Gehäuseteil 13 des Insertionskopfes 12 den verschieblich gelagerten Schieber 7, welcher den Federarm 20 mit der daran gebildeten Rastnase 10 trägt, mit und verschiebt diesen soweit nach rechts, dass die Rastnase 10 seitlich neben dem Hammerelement 9 positioniert wird und dieses nun ausschliesslich über den verriegelten ersten Arm 15a des Auslöserhebels entgegen der Kraft der Feder 8 in der vorgespannten Position gehalten wird. Diese Situation, in der die Insertionsvorrichtung applikationsbereit ist, ist in Fig. 5 dargestellt.

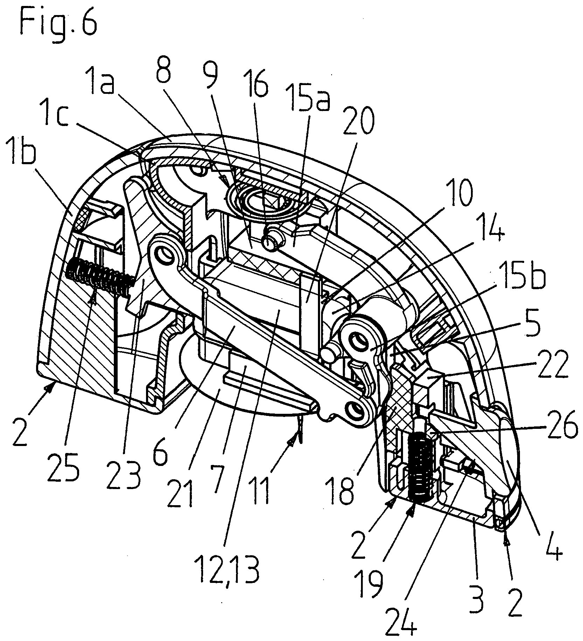

Wie aus einer Zusammenschau der Figuren 5 und 6 hervorgeht, welche die Insertionsvorrichtung jeweils im applikationsbereiten Zustand zeigen, jedoch einmal gesichert (Fig. 5) und einmal entsichert (Fig. 6), bildet der Sicherungstaster 3, auf welchem sich die Verriegelungsfeder 19 mit ihrem dem Riegelelement 18 abgewandten Ende abstützt, im ersten Gehäuseteil 1a einen Sicherungsschieber 22, welcher in der in Fig. 5 gezeigten Situation eine Betätigbarkeit des Auslöseknopfes 4 formschlüssig verhindert. Erst durch Beaufschlagung des Sicherungstasters 3 mit einer Druckkraft entgegen der Kraftrichtung der Verriegelungsfeder 19, z.B. durch Anpressen der Insertionsvorrichtung an die Applikationsstelle am Körper eines Patienten, kann dieser soweit in das Gehäuse 1a hineingeschoben werden, dass seine Unterseite, welche eine Kontaktfläche 2 trägt, im wesentlichen oberflächenbündig mit der an diese angrenzenden Kontaktfläche 2 des ersten Gehäuseteils 1a ist. In dieser Position, welche in Fig. 6 dargestellt ist, gibt der Sicherungsschieber 22 den Auslöseknopf 4 frei.

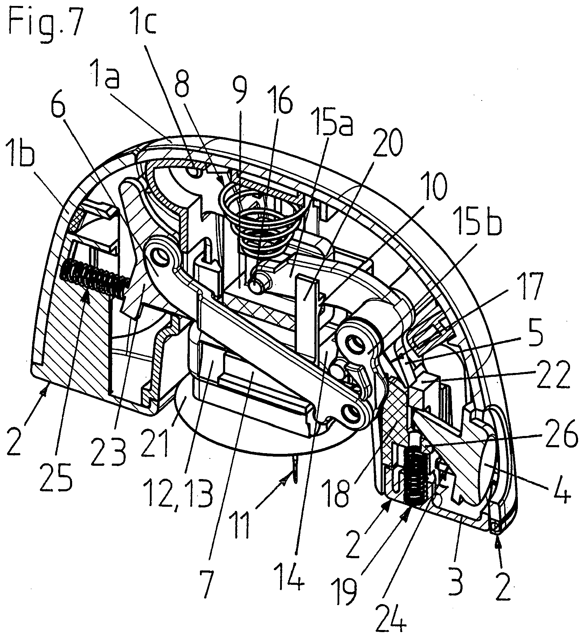

Wie aus Fig. 7 hervorgeht, welche die Insertionsvorrichtung kurz nach dem Betätigen des Auslöseknopfes 4 zeigt, weist der Auslöseknopf 4 auf seiner dem Inneren des Gehäuses 1a zugewandten Seite eine Auslöserampe 24 auf, welche beim Betätigen des Auslöseknopfes 4 entlang einer Steuernase 26 des Riegelelements 18 bewegt wird und dieses dadurch entgegen der Kraft der Verriegelungsfeder 19 nach unten zieht, wodurch der Rastvorsprung 5 des Auslöserriegels 15a, 15b freigegeben wird und das an dessen erstem Arm 15a gehaltene Hammerelement 9 durch die Kraft der vorgespannten Spiralfeder 8 getrieben nach unten schnellt. Dabei trifft das Hammerelement 9 auf die Oberseite des kraftschlüssig in der anspruchsgemässen ersten Position in den Halteelementen gehaltenen Insertionskopfes 12, löst diesen von den Halteelementen und treibt diesen, beim Applizieren des Insertionskopfes 12 am Körper eines Patienten, unter einem Einstechen der Kanüle 11 in den Körper weiter vor sich her, bis die Kanüle 11 vollständig eingestochen und der Insertionskopf 12 mit seiner Unterseite auf der Körperoberfläche aufliegt. Dabei verbleiben die Halteelemente in Insertionsrichtung unbewegt gegenüber dem Gehäuse 1a, 1b. Der Schieber 7 bewegt sich anschliessend in horizontaler Richtung durch die Kraft der Rückstellfeder getrieben wieder in die in den Figuren 1 bis 3 gezeigte Position.

Nach dem Applizieren ist der applizierte Insertionskopf 12 vollständig von der Insertionsvorrichtung getrennt, so dass diese ohne jegliche Irritation der Applikationsstelle entfernt werden kann. Sie befindet sich dann wieder in der in Fig. 1 dargestellten Situation und kann erneut für die Applikation einer Insertionskopfes verwendet werden.

## Patentansprüche

1. Insertionsvorrichtung zur Applikation eines Insertionskopfs (12), welcher mindestens eine Infusionskanüle (11) und/ oder mindestens eine Einstechspitze zum Einstechen in den Körper eines Patienten aufweist,
die Insertionsvorrichtung umfassend
a) ein Gehäuse (1a, 1b) mit mindestens einer Kontaktfläche (2) zum Aufsetzen der Insertionsvorrichtung auf eine Applikationsstelle am Körper des Patienten,
wobei das Gehäuse (1a, 1 b) einen ersten Gehäuseteil (1a) und einen zweiten Gehäuseteil (1b) aufweist,
und wobei der erste Gehäuseteil (1a) zumindest einen Teil der mindesten einen Kontaktfläche (2) trägt
und der zweite Gehäuseteil (1 b) gegenüber dem ersten Gehäuseteil (1a) von einer Grundposition in eine Ladeposition schwenkbar oder verschiebbar ist
und wobei die beiden Gehäuseteile (1a, 1 b) in der Grundposition in einer relativen Position zueinander angeordnet sind, welche sie während der Applikation des Insertionskopfs (12) einnehmen;
b) vom ersten Gehäuseteil (1a) getragene Haltemittel, welche dazu ausgelegt sind, den Insertionskopf (12) vorübergehend applikationsbereit in den Haltemitteln in einer ersten Position zu halten, in welcher sämtliche der mindestens einen Infusionskanüle (11) und/oder der mindestens einen Einstechspitze gegenüber der Kontaktfläche (2) zurückstehen,
wobei die Haltemittel in der Ladeposition zum Einsetzen des zu applizierenden Insertionskopfs (12) in die Haltemittel zugänglich sind;
und wobei der zweite Gehäuseteil (1 b) nach dem Einsetzten des Insertionskopfes in die Haltemittel zurück in die Grundposition bewegbar ist
c) Antriebsmittel (8, 9), welche dazu ausgelegt sind, den in den Haltemitteln in der ersten Position gehaltenen Insertionskopf (12) mittels einer Insertionsbewegung von der ersten Position in eine zweite Position zu bewegen,
in welcher sämtliche der mindestens einen Infusionskanüle (11) und/oder der mindestens einen Einstechspitze im wesentlichen vollständig über die Kontaktfläche (2) hinaus stehen;
d) ein von den Antriebsmitteln (8, 9) umfasstes Vortriebselement (9), welches dazu ausgelegt ist, den Insertionskopf zu Beginn der Insertionsbewegung aus den Haltemitteln zu schlagen und dadurch von diesen zu lösen,
wodurch der Insertionskopf (12) zumindest den grössten Teil der Insertionsbewegung losgelöst von den Haltemitteln vollführt.

2. Insertionsvorrichtung nach Anspruch 1, **dadurch gekennzeichnet, dass** die Insertionsvorrichtung derartig ausgebildet ist, dass der Insertionskopf (12) bei in der Grundposition angeordneten Haltemitteln in der ersten Position rein kraftschlüssig, rein formschlüssig oder kraft- und formschlüssig von den Haltemitteln gehalten werden kann.

3. Insertionsvorrichtung nach Anspruch 2, **dadurch gekennzeichnet, dass** die Insertionsvorrichtung derartig ausgebildet ist, dass der Insertionskopf (12) bei in der Ladeposition angeordneten Haltemitteln rein formschlüssig oder kraft- und formschlüssig von den Haltemitteln gehalten werden kann.

4. Insertionsvorrichtung nach Anspruch 3, **dadurch gekennzeichnet, dass** der Formschluss beim Bewegen des zweiten Gehäuseteils (1 b) von der Ladeposition in die Grundposition aufgehoben wird.

5. Insertionsvorrichtung nach Anspruch 2, **dadurch gekennzeichnet, dass** die Insertionsvorrichtung derartig ausgebildet ist, dass der Insertionskopf (12) bei in der Ladeposition angeordneten Haltemitteln rein kraftschlüssig von den Haltemitteln gehalten werden kann.

6. Ansertionsvorrichtung nach Anspruch 5, **dadurch gekennzeichnet, dass** der Kraftschluss beim Bewegen des zweiten Gehäuseteils (1b) von der Ladeposition in die Grundposition verringert wird

7. Insertionsvorrichtung nach einem der vorangehenden Ansprüche, **dadurch gekennzeichnet, dass** die Insertionsvorrichtung derartig ausgebildet ist, dass die Haltemittel zumindest während eines Grossteils der Insertionsbewegung oder während der gesamten Insertionsbewegung des Insertionskopfes (12) unbewegt gegenüber der Kontaktfläche (2) sind.

8. Insertionsvorrichtung nach Anspruch 7, **dadurch gekennzeichnet, dass** die Haltemittel unbeweglich gegenüber dem Teil der Kontaktflächen (2) sind, welcher vom ersten Gehäuseteil (1 b) getragen ist.

9. Insertionsvorrichtung nach einem der vorangehenden Ansprüche, **dadurch gekennzeichnet, dass** die Haltemittel zum kraftschlüssigen Halten des Insertionskopfes (12) in der ersten Position ausgebildet sind.

10. Insertionsvorrichtung nach einem der vorangehenden Ansprüche, **dadurch gekennzeichnet, dass** die Antriebsmittel (8, 9) mindestens ein vorspannbares Kraftspeicherelement (8) zur Bereitstellung der Antriebsenergie für die Insertionsbewegung aufweisen, insbesondere eine Spiral-, Schenkel- oder Blattfeder aus Metall oder Kunststoff, eine Gasdruckfeder oder ein Gummifederelement.

11. Insertionsvorrichtung nach Anspruch 10, **dadurch gekennzeichnet, dass** das Kraftspeicherelement (8) durch das Bewegen des zweiten Gehäuseteils (1b) von der Grundposition in die Ladeposition vorspannbar ist.

12. Insertionsvorrichtung nach einem der Ansprüche 10 bis 11, **dadurch gekennzeichnet, dass** das Kraftspeicherelement (8) durch das Bewegen des zweiten Gehäuseteils (1b) von der Ladeposition in die Grundposition vorspannbar ist.

13. Insertionsvorrichtung nach einem der Ansprüche 10 bis 12, **dadurch gekennzeichnet, dass** die Insertionsvorrichtung derartig ausgestaltet ist, dass das Kraftspeicherelement (8) bei in der ersten Position befindlichem Insertionskopf (12) im vorgespannten Zustand bereitstellbar ist und sodann die Insertionsbewegung durch Betätigen eines Betätigungsorgans (3, 4) unter zunehmender Entspannung des Kraftspeicherelements (8) auslösbar ist.

14. Insertionsvorrichtung nach Anspruch 13, **dadurch gekennzeichnet, dass** die Antriebsmittel (8, 9) derartig ausgestaltet sind, dass das Kraftspeicherelement (8) unter einem Verschieben des Vortriebselements (9) entgegen der Richtung der Insertionsbewegung und unter anschliessendem Verrasten desselben mit durch die Betätigungsorgane (3, 4) lösbaren Rastmitteln (5, 18) vorspannbar und in vorgespanntem Zustand bereitstellbar ist.

15. Insertionsvorrichtung nach einem der vorangehenden Ansprüche, **dadurch gekennzeichnet, dass** der zweite Gehäuseteil (1b) gegenüber dem ersten Gehäuseteil (1a) zum Bewegen von der Grundposition in die Ladeposition quer, insbesondere senkrecht zur Richtung der Insertionsbewegung verschiebbar ist.

16. Insertionsvorrichtung nach einem der vorangehenden Ansprüche, **dadurch gekennzeichnet, dass** der zweite Gehäuseteil (1b) einen Teil der Kontaktflächen (2) trägt.

17. Insertionsvorrichtung nach einem der vorangehenden Ansprüche, **dadurch gekennzeichnet, dass** die Haltemittel derartig ausgebildet sind, dass der Insertionskopf (12) in der Ladeposition durch ein Einschieben in einer Richtung quer, insbesondere senkrecht zur Richtung der Insertionsbewegung in diese eingesetzt werden kann.

18. Insertionsvorrichtung nach einem der vorangehenden Ansprüche, **dadurch gekennzeichnet, dass** die Insertionsvorrichtung mindestens zwei Betätigungsorgane (3, 4) aufweist, welche zur Auslösung der Insertionsbewegung gleichzeitig betätigt sein müssen, wobei insbesondere ein erstes der Betätigungsorgane (3) derartig ausgebildet ist, dass es durch ein Andrücken der Insertionsvorrichtung mit der Kontaktfläche (2) an den Körper des Patienten betätigbar ist.

19. Insertionsvorrichtung nach Anspruch 18, **dadurch gekennzeichnet, dass** das erste Betätigungsorgan (3) als schieber- oder tasterförmiges Element (3) ausgebildet ist, welches insbesondere sämtliche Kontaktflächen (2) bildet.

20. Insertionsvorrichtung nach einem der Ansprüche 18 bis 19, **dadurch gekennzeichnet, dass** ein zweites Betätigungsorgan (4) als tasten- oder knopfförmiges Element (4) ausgebildet ist, welches durch Druckbeaufschlagung mit einer Fingerkuppe des Bedieners betätigbar ist.

21. Insertionsvorrichtung nach einem der Ansprüche 18 bis 20, **dadurch gekennzeichnet, dass** die mindestens zwei Betätigungsorgane (3, 4) derartig miteinander wirkverbunden sind, dass durch die Betätigung des einen Betätigungsorgans (3) eine Sperrung des anderen Betätigungsorgans (4) aufhebbar ist.

22. Insertionsvorrichtung nach einem der vorangehenden Ansprüche mit mindestens einem Betätigungsorgan (3, 4) zum Auslösen der Insertionsbewegung, **dadurch gekennzeichnet, dass** das Betätigungsorgan oder die Betätigungsorgane (3, 4) zur Ermöglichung eines einhändigen Auslösens der Insertionsbewegung derartig ausgebildet sind, dass diese mit einer Hand betätigbar sind.

23. Insertionsvorrichtung nach einem der vorangehenden Ansprüche mit mindestens einem Betätigungsorgan (3, 4) zum Auslösen der Insertionsbewegung, **dadurch gekennzeichnet, dass** das Betätigungsorgan oder die Betätigungsorgane (3, 4) derartig ausgebildet sind, dass sie bei Wegfall einer Betätigungskraft automatisch wieder in einen unbetätigten Zustand übergehen.

24. Insertionsvorrichtung nach einem der vorangehenden Ansprüche, **dadurch gekennzeichnet, dass** die Haltemittel zum Halten eines Insertionskopfes (12) ausgelegt sind, welcher ein verschiebliches Aktivierungsorgan (13) und mindestens ein ausklappbare Infusionskanüle (11) und/oder mindestens eine ausklappbare Einstechspitze aufweist,
und die Insertionsvorrichtung Mittel (23) zur Bewirkung einer Verschiebung des Aktivierungsorgans (13) während dem Bewegen des zweiten Gehäuseteils (1 b) von der Ladeposition in die Grundposition aufweist,
zur Ermöglichung eines automatischen Ausklappens sämtlicher ausklappbaren Infusionskanülen (11) und Einstechspitzen des Insertionskopfes (12) während dem Bewegen des zweiten Gehäuseteils (1 b) von der Ladeposition in die Grundposition.

25. Insertionsvorrichtung nach Anspruch 24, **dadurch gekennzeichnet, dass** die Mittel (23) zur Bewirkung einer Verschiebung des verschieblichen Aktivierungsorgans (13) eine vom zweiten Gehäuseteil (1b) gebildet Anlauffläche zum Bewirken der Verschiebung des Aktivierungsorgan (13) umfassen.

26. Insertionsvorrichtung nach einem der Ansprüche 24 bis 25, **dadurch gekennzeichnet, dass** die Mittel (23) zur Bewirkung einer Verschiebung des verschieblichen Aktivierungsorgans (13) einen Hebelmechanismus (23) umfassen, mit welchem das verschiebliches Aktivierungsorgan (13) verschoben werden kann.

27. Insertionsvorrichtung nach einem der vorangehenden Ansprüche, **dadurch gekennzeichnet, dass** die Insertionsvorrichtung derartig ausgestaltet ist, dass sie mehrfach zum Applizieren eines Insertionskopfes (12) verwendet werden kann.

28. Anordnung umfassend eine Insertionsvorrichtung nach einem der vorangehenden Ansprüche und einen in dieser aufgenommenen oder aufnehmbaren Insertionskopf (2) mit mindestens einer Infusionskanüle (11) und/oder mindestens einer Einstechspitze.

29. Anordnung nach Anspruch 28, **dadurch gekennzeichnet, dass** der Insertionskopf (12) mindestens eine ausklappbare Infusionskanüle (11) und/oder mindestens eine ausklappbare Einstechspitze aufweist, wobei sämtliche ausklappbaren Infusionskanülen (11) und Einstechspitzen des Insertionskopfes (12) im nicht-aktivierten Zustand, in welchem der Insertionskopf (12) bei in der Ladeposition befindlichem zweiten Gehäuseteil (1 b) in den Haltemitteln aufgenommen ist, eingeklappt sind und die Insertionsvorrichtung und der Insertionskopf (12) derartig ausgebildet sind, dass sämtliche ausklappbaren Infusionskanülen (11) und Einstechspitzen des Insertionskopfes (12) beim Bewegen des zweiten Gehäuseteils (1b) von der Ladeposition in die Grundposition automatisch ausgeklappt werden.

## Claims

1. An insertion device for application of an insertion head (12), which has at least one infusion cannula (11) and/or at least one piercing tip for piercing into the body of a patient, the insertion device comprising
a) a housing (1a, 1b) having at least one contact surface (2) for placing the insertion device on an application point on the body of the patient,
wherein the housing (1a, 1b) has a first housing part (1a) and a second housing part (1b),
and wherein the first housing part (1a) carries at least a part of the at least one contact surface (2),
and the second housing part (1b) is pivotable or displaceable in relation to the first housing part (1a) from a base position into a charging position,
and wherein the two housing parts (1a, 1b) are arranged in the base position in a relative position in relation to one another which they assume during the application of the insertion head (12);
b) holding means supported by the first housing part (1a) which are designed for the purpose of temporarily holding the insertion head (12) ready for application in the holding means in a first position, in which both of the at least one infusion cannula (11) and/or the at least one piercing tip are set back in relation to the contact surface (2),
wherein the holding means are accessible in the charging position for insertion of the insertion head (12) to be applied into the folding means;
and wherein the second housing part (1b) is moveable back into the base position after the insertion of the insertion head into the holding means;
c) drive means (8, 9) which are designed to move the insertion head (12), which is held in the holding means in the first position, by means of an insertion movement from the first position into a second position,
in which both of the at least one infusion cannula (11) and/or the at least one piercing tip protrude substantially completely beyond the contact surface (2);
d) a propulsion element (9) comprised by the drive means (8, 9), which is designed to strike the insertion head out of the holding means at the beginning of the insertion movement and thus detach it therefrom,
whereby the insertion head (12) completes at least the largest part of the insertion movement detached from the holding means.

2. The insertion device as claimed in claim 1, **characterized in that** the insertion device is designed such that the insertion head (12), when the holding means is arranged in the base position, can be held in the first position by the holding means solely by friction locking, solely by a form fit, or by friction locking and a form fit.

3. The insertion device as claimed in claim 2, **characterized in that** the insertion device is designed such that the insertion head (12), when the holding means is arranged in the charging position, can be held by the holding means solely by a form fit or by friction locking and a form fit.

4. The insertion device as claimed in claim 3, **characterized in that** the form fit is overturned during the movement of the second housing part (1b) from the charging position into the base position.

5. The insertion device as claimed in claim 2, **characterized in that** the insertion device is designed such that the insertion head (12), when the holding means are arranged in the charging position, can be held by the holding means solely by friction locking.

6. The insertion device as claimed in claim 5, **characterized in that** the friction lock is reduced upon the movement of the second housing part (1b) from the charging position into the base position.

7. The insertion device as claimed in any one of the preceding claims, **characterized in that** the insertion device is designed such that the holding means are unmoved in relation to the contact surface (2) at least during a majority of the insertion movement or during the entire insertion movement of the insertion head (12).

8. The insertion device as claimed in claim 7, **characterized in that** the holding means is immovable in relation to the part of the contact surfaces (2) which is carried by the first housing part (1b).

9. The insertion device as claimed in any one of the preceding claims, **characterized in that** the holding means are designed for friction-locked holding of the insertion head (12) in the first position.

10. The insertion device as claimed in any one of the preceding claims, **characterized in that** the drive means (8, 9) have at least one force accumulator element (8), which can be pretensioned, for providing the drive energy for the insertion movement, in particular a coiled, leg, or leaf spring made of metal or plastic, a gas pressure spring, or a rubber spring element.

11. The insertion device as claimed in claim 10, **characterized in that** the force accumulator element (8) can be pretensioned by the movement of the second housing part (1b) from the base position into the charging position.

12. The insertion device as claimed in any one of claims 10 to 11, **characterized in that** the force accumulator element (8) can be pretensioned by the movement of the second housing part (1b) from the charging position into the base position.

13. The insertion device as claimed in any one of claims 10 to 12, **characterized in that** the insertion device is designed such that the force accumulator element (8) can be provided in the pretensioned state when the insertion head (12) is located in the first position, and then the insertion movement can be triggered by actuating an actuating element (3, 4) with increasing relaxation of the force accumulator element (8).

14. The insertion device as claimed in claim 13, **characterized in that** the drive means (8, 9) are designed such that the force accumulator element (8) can be pretensioned by displacing of the propulsion element (9) opposite to the direction of the insertion movement and by subsequently locking said element with catch means (5, 18), which can be triggered by the actuating elements (3, 4), and can be provided in the pretensioned state.

15. The insertion device as claimed in any one of the preceding claims, **characterized in that** the second housing part (1b) is displaceable transversely, in particular perpendicularly, in relation to the direction of the insertion movement, in relation to the first housing part (1a) for the movement from the base position into the charging position.

16. The insertion device as claimed in any one of the preceding claims, **characterized in that** the second housing part (1b) carries a part of the contact surfaces (2).

17. The insertion device as claimed in any one of the preceding claims, **characterized in that** the holding means is designed such that the insertion head (12) can be inserted therein in the charging position by an insertion in a direction transversely, in particular perpendicularly in relation to the direction of the insertion movement.

18. The insertion device as claimed in any one of the preceding claims, **characterized in that** the insertion device has at least two actuating elements (3, 4), which have to be actuated simultaneously to trigger the insertion movement, wherein in particular a first of the actuating elements (3) is designed such that it can be actuated by pressing the insertion device with the contact surface (2) against the body of the patient.

19. The insertion device as claimed in claim 18, **characterized in that** the first actuating element (3) is designed as an element (3) in the form of a slide or button, which in particular forms all contact surfaces (2).

20. The insertion device as claimed in any one of claims 18 to 19, **characterized in that** a second actuating element (4) is designed as an element (4) in the form of a button or knob, which can be actuated by application of pressure using a fingertip of an operator.

21. The insertion device as claimed in any one of claims 18 to 20, **characterized in that** the at least two actuating elements (3, 4) are operationally connected to one another such that a blocking of the other actuating element (4) can be removed by the actuation of one actuating element (3).

22. The insertion device as claimed in any one of the preceding claims having at least one actuating element (3, 4) for triggering the insertion movement, **characterized in that** the actuating element or the actuating elements (3, 4), to enable one-handed triggering of the insertion movement, are designed such that they can be actuated with one hand.

23. The insertion device as claimed in any one of the preceding claims having at least one actuating element (3, 4) for triggering the insertion movement, **characterized in that** the actuating element or the actuating elements (3, 4) are designed such that they automatically move back into a non-actuated state when an actuating force is discontinued.

24. The insertion device as claimed in any one of the preceding claims, **characterized in that** the holding means is designed for holding an insertion head (12), which has a displaceable activation element (13) and at least one extendable infusion cannula (11) and/or at least one extendable piercing tip,
and the insertion device has means (23) for causing a displacement of the activation elements (13) during the movement of the second housing part (1b) from the charging position into the base position,
to enable automatic extension of all extendable infusion cannulas (11) and piercing tips of the insertion head (12) during the movement of the second housing part (1b) from the charging position into the base position.

25. The insertion device as claimed in claim 24, **characterized in that** the means (23) for causing a displacement of the displaceable activation element (13) comprise a thrust surface, which is formed by the second housing part (1b), for causing the displacement of the activation element (13).

26. The insertion device as claimed in any one of claims 24 to 25, **characterized in that** the means (23) for causing a displacement of the displaceable activation element (13) comprises a lever mechanism (23), which can displace the displaceable activation element (13).

27. The insertion device as claimed in any one of the preceding claims, **characterized in that** the insertion device is designed such that it can be used multiple times for applying an insertion head (12).

28. An arrangement comprising an insertion device according to any one of the preceding claims and an insertion head (2), which is accommodated or can be accommodated therein, having at least one infusion cannula (11) and/or at least one piercing tip.

29. The arrangement as claimed in claim 28, **characterized in that** the insertion head (12) has at least one extendable infusion cannula (11) and/or at least one extendable piercing tip, wherein all extendable infusion cannulas (11) and piercing tips of the insertion head (12), in the nonactivated state, in which the insertion head (12) is received in the holding means with the second housing part (1b) located in the charging position, are retracted and the insertion device and the insertion head (12) are designed such that all extendable infusion cannulas (11) and piercing tips of the insertion head (12) are automatically extended upon the movement of the second housing part (1b) from the charging position into the base position.

## Revendications

1. Dispositif d'insertion pour l'application d'une tête d'insertion (12) comportant au moins une canule d'insertion (11) et/ou au moins une pointe à piquer destinée à être piquée dans le corps d'un patient,
le dispositif d'insertion comprenant
a) un boîtier (1a, 1b) avec au moins une surface de contact (2) permettant de poser le dispositif d'insertion sur une zone d'application sur le corps du patient,
le (1a, 1b) comportant une première partie de boîtier (1a) et une deuxième partie de boîtier (1b),
et la première partie de boîtier (1a) portant au moins une partie de l'au moins une surface de contact (2),
et les deux parties de boîtier (1a, 1b) étant agencées dans une position relative l'une par rapport à l'autre dans la position de base, dans laquelle elles se mettent pendant l'application de la tête d'insertion (12) ;
b) des moyens de maintien portés par la première partie de boîtier (1a), lesquels sont conçus pour maintenir la tête d'insertion (12) prête à l'application temporairement dans les moyens de maintien dans une première position dans laquelle l'ensemble de l'au moins une canule d'insertion (11) et/ou de l'au moins une pointe à piquer sont en retrait par rapport à la surface de contact (2),
les moyens de maintien étant accessibles dans la position de chargement pour l'engagement de la tête d'insertion dans les moyens de maintien ;
et la deuxième partie de boîtier (1b) pouvant être remise dans la position de base après l'engagement de la tête d'insertion (12) dans les moyens de maintien,
c) des moyens d'entraînement (8, 9) conçus pour déplacer la tête d'insertion (12) maintenue dans la première position dans les moyens de maintien de la première position vers la deuxième position à l'aide d'un mouvement d'insertion,
dans laquelle l'ensemble de l'au moins une canule d'insertion (11) et/ou de l'au moins une pointe à piquer font saillie quasiment entièrement au-delà de la surface de contact (2) ;
d) un élément d'avancement (9) entouré par les moyens d'entraînement (8, 9), lequel est conçu pour faire sortir la tête d'insertion hors des moyens de maintien au début du mouvement d'insertion et ainsi la détacher de ceux-ci,
moyennant quoi la tête d'insertion (12) effectue au moins la majeure partie du mouvement d'insertion en étant détachée des moyens de maintien.

2. Dispositif d'insertion selon la revendication 1, **caractérisé en ce que** le dispositif d'insertion est conçu de telle façon que la tête d'insertion (12) peut être maintenue dans la première position par les moyens de maintien uniquement par la force, uniquement par complémentarité de forme ou par la force et par complémentarité de forme, lorsque les moyens de maintien sont placés dans la position de base.

3. Dispositif d'insertion selon la revendication 2, **caractérisé en ce que** le dispositif d'insertion est conçu de telle façon que la tête d'insertion (12) peut être maintenue par les moyens de maintien uniquement par complémentarité de forme ou par la force et par complémentarité de forme lorsque les moyens de maintien sont placés dans la position de chargement.

4. Dispositif d'insertion selon la revendication 3, **caractérisé en ce que** la complémentarité de forme est supprimée lors du déplacement de la deuxième partie de boîtier (1b) de la position de chargement vers la position de base.

5. Dispositif d'insertion selon la revendication 2, **caractérisé en ce que** le dispositif d'insertion est conçu de telle façon que la tête d'insertion (12) peut être maintenue par les moyens de maintien uniquement par la force lorsque les moyens de maintien sont placés dans la position de chargement.

6. Dispositif d'insertion selon la revendication 5, **caractérisé en ce que** l'assemblage par la force est réduit lors du déplacement de la deuxième partie de boîtier (1b) de la position de chargement vers la position de base.

7. Dispositif d'insertion selon l'une des revendications précédentes, **caractérisé en ce que** le dispositif d'insertion est conçu de telle façon que les moyens de maintien sont immobiles par rapport à la surface de contact (2) pendant une majeure partie du mouvement d'insertion ou pendant la totalité du mouvement d'insertion de la tête d'insertion (12).

8. Dispositif d'insertion selon la revendication 7, **caractérisé en ce que** les moyens de maintien sont immobiles par rapport à la partie des surfaces de contact (2) qui est portée par la première partie de boîtier (1b).

9. Dispositif d'insertion selon l'une des revendications précédentes, **caractérisé en ce que** les moyens de maintien sont conçus pour maintenir la tête d'insertion (12) par la force dans la première position.

10. Dispositif d'insertion selon l'une des revendications précédentes, **caractérisé en ce que** les moyens d'entraînement (8, 9) comportent au moins un élément de stockage de force (8) apte à être précontraint, pour l'apport de l'énergie d'entraînement nécessaire au mouvement d'insertion, en particulier un ressort hélicoïdal, un ressort à branches ou un ressort à lame en métal ou en plastique, un ressort à pression de gaz ou un élément élastique en caoutchouc.

11. Dispositif d'insertion selon la revendication 10, **caractérisé en ce que** l'élément de stockage de force (8) peut être précontraint de la position de base vers la position de chargement par le mouvement de la deuxième partie de boîtier (1b).

12. Dispositif d'insertion selon l'une des revendications 10 à 11, **caractérisé en ce que** l'élément de stockage de force (8) peut être précontraint de la position de chargement vers la position de base par le mouvement de la deuxième partie de boîtier (1b).

13. Dispositif d'insertion selon l'une des revendications 10 à 12, **caractérisé en ce que** le dispositif d'insertion est conçu de telle façon que l'élément de stockage de force (8) peut être mis à disposition dans l'état précontraint lorsque la tête d'insertion (12) se trouve dans la première position, et que le mouvement d'insertion peut ensuite être amorcé en actionnant un organe d'actionnement (3, 4) avec détente croissante de l'élément de stockage de force (8).

14. Dispositif d'insertion selon la revendication 13, **caractérisé en ce que** les moyens d'entraînement (8, 9) sont conçus de telle façon que l'élément de stockage de force (8) peut être précontraint et mis à disposition dans l'état précontraint en déplaçant l'élément d'avancement (9) contre la direction du mouvement d'insertion et en encliquetant ensuite celui-ci à l'aide de moyens d'encliquetage (5, 18) aptes à être libérés par les organes d'actionnement (3, 4).

15. Dispositif d'insertion selon l'une des revendications précédentes, **caractérisé en ce que** la deuxième partie de boîtier (1b) peut être déplacé transversalement, en particulier perpendiculairement à la direction du mouvement d'insertion, par rapport à la première partie de boîtier (1a), pour le déplacement de la position de base vers la position de chargement.

16. Dispositif d'insertion selon l'une des revendications précédentes, **caractérisé en ce que** la deuxième partie de boîtier (1b) porte une partie des surfaces de contact (2).

17. Dispositif d'insertion selon l'une des revendications précédentes, **caractérisé en ce que** les moyens de maintien sont conçus de telle façon que la tête d'insertion (12) dans la position de chargement peut être engagée dans ceux-ci par insertion dans une direction transversalement, en particulier perpendiculaire à la direction du mouvement d'insertion.

18. Dispositif d'insertion selon l'une des revendications précédentes, **caractérisé en ce que** le dispositif d'insertion comporte au moins deux organes d'actionnement (3, 4), lesquels sont censés être actionnés simultanément pour amorcer le mouvement d'insertion, un premier des organes d'actionnement (3) étant en particulier conçu de façon à pouvoir être actionné par pression du dispositif d'insertion avec la surface de contact (2) sur le corps du patient.

19. Dispositif d'insertion selon la revendication 18, **caractérisé en ce que** le premier organe d'actionnement (3) est conçu comme un élément en forme de poussoir ou de touche (3) formant en particulier toutes les surface de contact (2).

20. Dispositif d'insertion selon l'une des revendications 18 à 19, **caractérisé en ce qu'**un deuxième organe d'actionnement (4) est conçu comme un élément en forme de touche ou de bouton (4), lequel peut être actionné par pression avec le bout du doigt de l'opérateur.

21. Dispositif d'insertion selon l'une des revendications 18 à 20, **caractérisé en ce que** les au moins deux organes d'actionnement (3, 4) sont reliés fonctionnellement de telle façon que l'actionnement de l'un des organes d'actionnement (3) permet de supprimer un verrouillage de l'autre organe d'actionnement (4).

22. Dispositif d'insertion selon l'une des revendications précédentes, comprenant au moins un organe d'actionnement (3, 4) destiné à amorcer le mouvement d'insertion, **caractérisé en ce que** l'organe d'actionnement ou les organes d'actionnement (3, 4) sont conçus de manière à pouvoir être actionnés avec une seule main pour permettre l'amorce du mouvement d'insertion avec une seule main.

23. Dispositif d'insertion selon l'une des revendications précédentes, comprenant au moins un organe d'actionnement (3, 4) destiné à amorcer le mouvement d'insertion, **caractérisé en ce que** l'organe d'actionnement ou les organes d'actionnement (3, 4) sont conçus de façon à revenir automatiquement dans un état non actionné en l'absence d'une force d'actionnement.

24. Dispositif d'insertion selon l'une des revendications précédentes, **caractérisé en ce que** les moyens de maintien sont conçus pour maintenir une tête d'insertion (12) comportant un organe d'activation coulissant (13) et au moins une canule de perfusion rétractable (11) et/ou au moins une pointe à piquer rétractable,
et le dispositif d'insertion comporte des moyens (23) destinés à faire coulisser l'organe d'activation (13) pendant le déplacement de la deuxième partie de boîtier (1b) de la position de chargement vers la position de base,
pour permettre un déploiement automatique de l'ensemble des canules de perfusion rétractables (11) et des pointes à piquer de la tête d'insertion (12) pendant le déplacement de la deuxième partie de boîtier (1b) de la position de chargement vers la position de base.

25. Dispositif d'insertion selon la revendication 24, **caractérisé en ce que** les moyens (23) destinés à faire coulisser l'organe d'activation coulissant (13) comprennent une surface de butée formée par la deuxième partie de boîtier (1b) pour provoquer le déplacement de l'organe d'activation (13).

26. Dispositif d'insertion selon l'une des revendications 24 à 25, **caractérisé en ce que** les moyens (23) destinés à faire coulisser l'organe d'activation coulissant (13) comprennent un mécanisme de levier (23) permettant de faire coulisser l'organe d'activation coulissant (13).

27. Dispositif d'insertion selon l'une des revendications précédentes, **caractérisé en ce que** le dispositif d'insertion est conçu de manière à pouvoir être utilisé plusieurs fois pour l'application d'une tête d'insertion (12).

28. Agencement comprenant le dispositif d'insertion selon l'une des revendications précédentes, ainsi qu'une tête d'insertion (12) reçue ou apte à être reçue dans celui-ci, avec au moins une canule de perfusion (11) et/ou au moins une pointe à piquer.

29. Agencement selon la revendication 28, **caractérisé en ce que** la tête d'insertion (12) comporte au moins une canule de perfusion rétractable (11) et/ou au moins une pointe à piquer rétractable, dans lequel l'ensemble des canules de perfusion rétractables (11) et des pointes à piquer de la tête d'insertion (12) sont rétractées dans l'état non activé, dans lequel la tête d'insertion (12) est reçue dans les moyens de maintien tandis que la deuxième partie de boîtier (1b) se trouve dans la position de chargement, et le dispositif d'insertion et la tête d'insertion (12) sont conçus de telle façon que l'ensemble des canules de perfusion rétractables (11) et des pointes à piquer de la tête d'insertion (12) sont déployées automatiquement lors du déplacement de la deuxième partie de boîtier (1b) de la position de chargement vers la position de base.
